# EUROPEAN PATENT APPLICATION

(11) **EP 4 353 824 A1**
(43) Date of publication of application: **17.04.2024**
(21) Application number: 22201471.4
(22) Date of filing: 13.10.2022
(51) Int. Cl.: C12N 15/82, C07K 14/08

(54) **CONSTRUCT FOR INDUCING SILENCING IN PLANTS AND METHOD FOR PROTECTING THEM AGAINST TOMATO SPOTTED WILT VIRUS**

(30) Priority: 10.10.2022 PT 2022118250
(71) Applicant: Universidade de Évora, 7000-803 Évora (PT)
(72) Inventor: REIS VARANDA, CARLA MARISA, Évora (PT); DE QUEIROZ MATERATSKI, PATRICK JOSÉ, ÉVORA (PT); FERNANDES FÉLIX, MARIA DO ROSÁRIO, ÉVORA (PT); MURTEIRA RICO DA COSTA CAMPOS, MARIA DOROTEIA, ÉVORA (PT); CARDOSO PATANITA, MARIANA, BEJA (PT); BARRETO ALBUQUERQUE, ANDRÉ FILIPE, Évora (PT); AMARO RIBEIRO, JOANA, REGUENGOS DE MONSARAZ (PT)
(74) Representative: Pereira da Cruz, Joao

(57) **Abstract**

The invention relates to a viral vector for conferring protection of plants against *Tomato spotted wilt virus* (TSWV), a virus that causes a devastating disease in tomato plants. The virus-based vector was modified to carry part of the TSWV non-structural protein NSm gene, conferring protection against TSWV in plants. The invention also relates to a process for silencing a target gene in a plant comprising the steps of i) obtaining a construct according to the previous claim; ii) introducing the construct inside the plant, preferably by using viral suspensions; inoculating into plants, preferably by spraying plants, even more preferably by spraying plants with a compressor set comprising an airbrush; iii) activating the plant's specific defence mechanism, consequently silencing the target gene. The present invention also discloses a composition for plant disease management and/or plant immunization.

## Description

### Field of the Invention

The present invention is enclosed in the area of Genetic Engineering and Biotechnology.

The invention relates to a viral vector for conferring protection of plants against *Tomato spotted wilt virus* (TSWV), a virus that causes a devastating disease in tomato plants. The virus-based vector was modified to carry part of the TSWV non-structural protein NSm gene, conferring protection against TSWV in plants.

### Background of the Invention

Tomato spotted wilt virus (TSWV) is included in the European and Mediterranean Plant Protection Organization (EPPO) quarantine list. It infects over 271 species of plants (Edwarson & Christie 1986).

TSWV is well established in several areas worldwide, including Portugal. This virus belongs to genus Tospovirus and is transmitted by thrips, being the most efficient vector, *Frankliniella occidentalis,* which is also distributed worldwide. TSWV causes a major disease of tomato (*Solanum lycopersicum*) with very significant economic impacts.

Tomato is one of the most important crops in the world, in terms of economic value, tomato constitutes 72% of the value of fresh vegetables produced worldwide. In Portugal, tomato is the most competitive agro-industrial product, with productions above the European production average, 64.000 ton/ha.

TSWV symptoms in tomato include bronzing, curling, necrotic streaks and spots on the leaves and dark-brown streaks on stems. Infected plants are smaller and stunted when compared to healthy ones. The ripe fruits show paler red or yellow areas on the skin. In the most severe cases, affected plants die with severe necrosis.

Because it is one of the viruses with the widest host ranges and it is worldwide distributed, as well as its most efficient vector *Frankliniella occidentalis,* TSWV is very difficult to control and has been a major concern among producers.

Some solutions exist in the art to control this disease. Document EP4029370A1 discloses a lettuce plant (*Lactuca sativa L*.), comprising a genetic determinant, which confers resistance to tomato spotted wilt virus (TSWV) and/or impatiens necrotic spot virus (INSV), and which is as found in plants grown from seeds of which a representative sample was deposited under NCIMB accession number NCIMB 42023. The invention further provides seeds, progeny, propagation material and food products from the plant.

Document EP3082403A1 discloses an isolated nucleotide sequence and its use for marker assisted selection of plants. In addition, the invention regards the use of a nucleotide sequence for introgression into plants. Furthermore, the invention is directed to a method to make plants resistant to infections by TSWV strains harmful to plants having the Sw-5b allele, by introducing into the plant genome a nucleotide sequence.

Document TW200826836A discloses a method of using plant transgenics realized through tomato spotted wilt virus (TSWV) (tospovirus genus) RNA duplication high retention region to provide anti-TSWV effect and an application thereof, wherein replicase high retention region of watermelon silver spot virus is used for breeding and selecting transgenic plant with extensive resistance, whereby the transgenic plant so provided can simultaneously resist at least five kinds of different and genetically distant TSWV effectively.

Current most efficient control measures rely on controlling insect vectors and on the use of more tolerant varieties. Tolerant varieties however force producers to use a plant variety that does not present the features that producers desire in terms of colour, brix, production cycle, cost, etc. In addition, the continuous resurgence and spread of TSWV into new hosts make essential innovative and efficient means of control.

Until date, the best control strategy for a viral disease is the introduction of genetic resistance in the plant host. However, the continuous increase in the number of varieties of tomato makes that goal almost impossible.

The present solution intended to innovatively overcome such issues and provide a unique solution to silence the disease, avoiding the existence of an infection of the plant.

### General Description

It is therefore an object of the present invention to provide an effective method and product for protecting plants against TSWV.

The present invention comprises a vector, plant virus-based, modified to carry and introduce a partial sequence of the NSm TSWV gene in plants, conferring them protection against TSWV, being capable of replicating in several plants and that is highly efficient in silencing, acting as a Virus-induced gene silencing (VIGS) vector.

In an embodiment, the construct carries a partial TSWV sequence in antisense orientation and is placed inside tomato plants without causing in them any viral symptoms. This will activate plant silencing, plants will recognize the foreign sequences and activate their specific defence mechanism, giving them a significant advantage in its protection against a possible later infection of that virus.

The construct of SEQ ID Nº 3 is also called WiltVirusFree vector. This construct is stable, it replicates and accumulates in tomato plants, causing no disease symptoms and is able to induce silencing that allow conferring protection of plants against TSWV. This has a significant interest in that no antiviral products are available for plant disease management, leaving eradication or prevention through sanitary measures as the only immediate control strategies.

In an embodiment, the present invention discloses an isolated nucleic acid sequence having at least 95% homology with SEQ ID No 1, preferably at least 98% homology, even more preferably having at least 99% homology, the isolated sequence comprising at least one point mutation selected from: cytosine to guanine at nt 3361, guanine to cytosine at nt 3365 and/or guanine to thymine at nt 3368.

In a further embodiment, the present invention discloses an isolated sequence wherein the sequence is a partial sequence of the non-structural protein NSm from Tomato Spotted Wilt Virus (TSWV).

In a further embodiment, the present invention discloses a vector comprising the isolated sequence of the present invention.

In a further embodiment, the present invention discloses a construct comprising the vector according to the present invention and a partial isolated sequence of TSWV in antisense orientation.

In a further embodiment, the present invention discloses a construct comprising the vector of the present invention and an isolated sequence having at least 95% homology with SEQ ID No 2, preferably at least 98% homology, even more preferably having at least 99% homology with SEQ ID No 2.

In a further embodiment, the present invention discloses a construct according to the present invention comprising an isolated sequence having at least 95% homology with SEQ ID No 3, preferably at least 98% homology, even more preferably having at least 99% homology with SEQ ID No 3.

In a further embodiment, the present invention discloses a process for silencing a target gene in a plant comprising the steps of:
i) obtaining a vector or a construct of any of the embodiments of the present invention;
ii) introducing said vector or construct inside the plant, preferably by using viral suspension;
iii) inoculating the vector or construct into the plant, preferably by spraying, even more preferably by spraying with a compressor comprising an airbrush; consequently activating the plant's specific defence mechanism, therefore silencing the target gene.

In a further embodiment, the present invention discloses a process according to the previous embodiment wherein the plant is *Solanum lycopersicum* or *Latuca sativa L*..

In a further embodiment, the present invention discloses a process for producing and maintaining the vector of the present invention in *Nicotiana benthamiana.*

In a further embodiment, the present invention discloses a plant comprising a sequence with at least 95% homology, preferably at least 98% homology, even more preferably at least 99% homology with SEQ ID No.1, SEQ ID No 2, SEQ ID No 3 or a combination thereof, wherein the plant is not obtained exclusively by crossing and selection.

In a further embodiment, the present invention discloses a plant, wherein the plant is an edible berry of the plant *Solanum lycopersicum* or *Latuca sativa L..*

In a further embodiment, the present invention discloses a composition, particularly a phytopharmaceutical composition, for plant treatment comprising the sequence, vector or construct of the present invention.

In a further embodiment, the present invention discloses a composition of the previous claim for Plant disease management and/or Plant immunization.

### Detailed Description of the Invention

The more general and advantageous configurations of the present invention are described above. Such configurations are detailed below in accordance with other advantageous and/or preferred embodiments of implementation of the present invention.

The virus vector of the present invention (SEQ ID No 1) is based on Olive mild mosaic virus (OMMV), a virus originally isolated from olive (Cardoso et al., 2005). An infectious transcript of OMMV cDNA clone obtained from OMMV type strain (Accession number HQ651834.1) was modified to be non-fungal transmitted, through a single mutation from adenine to thymine at nucleotide 3200 resulting in a nonsynonymous change from asparagine to tyrosine at position 189 of the coat protein

(CP) (Varanda et al., 2011). This virus mutant was further modified to cause no disease symptoms in plants, through three point mutations: cytosine to guanine at nucleotide (nt) 3361 (proline to alanine at position 242 of CP aa sequence); guanine to cytosine at nt 3365 (alanine to proline at position 244 of CP aa sequence) and guanine to thymine at nt 3368 (alanine to serine at position 245 of CP aminoacid sequence. This new virus mutant was named OMMV_noSnoT.

The 3' sequence of the NSm gene of TSWV (SEQ ID No 2) (Accession number S48091.1, nucleotide 679 to 1009) 331 nt in length, was amplified through Polymerase chain reaction using primers NSm679_5' (5' AAACTGTTGTCAGCTGCATT 3') and NSm1009_3' (5' TATCCTTTGATGAAATATAG 3') and the KOD Hot Start DNA Polymerase that inhibits the 3' - 5' exonuclease activity, producing blunt-ended DNA products.

To obtain the construct of SEQ ID No. 3, the isolated sequence of SEQ ID No 2 was then ligated with T4 DNA ligase into OMMV_noSnoT, previously digested with the restriction enzyme HPal, which cuts in the position nt3454, 9 nt downstream of the CP and leaves blunt ends. Sequencing was performed to select constructs carrying TSWV sequence in antisense orientation.

The vector presented, consisting of OMMV_noSnoT (SEQ ID No 1) carrying a partial sequence of NSm TSWV gene (SEQ ID No2), is here termed WiltVirusFree vector (SEQ ID No. 3).

In an embodiment, the virus-based vector for conferring protection of plants against TSWV herein disclosed, is produced and maintained in *Nicotiana benthamiana* plants.

In an embodiment, macerated leaves are subjected to viral purification as described in Varanda et al., 2011. This step provides the technical advantages of Varanda et al., 2011, which is, particularly in what refers to the technical effects disclosed, are herein incorporated by reference. These technical effects are effectively surprising when applied to the technical problem solved by the present invention.

In an embodiment, viral suspensions are used to inoculate plants, for example by spraying, after loading the mixture into an airbrush attached to a compressor set which will be used to spray plant leaves. This step provides the technical advantage of achieving a more uniform inoculation of the plant having as a consequence a very high percentage of silencing of the target gene and the plant being more resistant to TSWV (results not shown).

### Example 1

Mutations performed to create OMMV_noSnoT were introduced using the QuikChange Site-Directed Mutagenesis kit Stratagene, in accordance to the instruction manual.

Primers were designed according to the primer design guidelines and were as follows: Mut_OMMV_Fwd: 5' GCTGGGGACGGCGGAGCTGTGCCTTCCACAGCTGTGGGC 3' and Mut_OMMV_Rev: 5' GCCCACAGCTGTGGAAGGCACAGCTCCGCCGTCCCCAGC; mutations are underlined. Mutation strand synthesis reaction was, for a final volume of 50 µL, as follows: 5 µL of 10x reaction buffer, 50 ng of plasmid DNA (OMMV in puC18), 125 ng of Mut_OMMV_Fwd and 125 ng of Mut_OMMV_Rev, 1 µL of dNTP mix and 2,5 U of PfuTurbo DNA polymerase. Cycling parameters were: 1 cycle at 95 °C for 30 seconds; 18 cycles of 95 °C at for seconds, 55 °C for 1 minute and 68 °C for 6 minutes. Reaction was placed on ice for 2 minutes to cool the reaction. Digestion of amplification products was performed through Dpn I digestion to digest the parental dsDNA. 1 µL Dpn I restriction enzyme was added to the reaction and incubated at 37 °C for 1 hour. Transformation was performed using XL1-Blue Supercompetent cells following the manufacturer's instructions.

The amplification reaction for the NSm partial gene consisted of 50 µL of: 1X buffer for KOD Start DNA Polymerase, 1,5 mM of MgSO₄, 0,2 mM of each dNTP, 0,3 µM of primer Anafp5', 0,3 µL of primer Anafp3', 1U KOD Hot Start DNA Polymerase and 10 ng of plasmid DNA of TSWV. Cycling conditions consisted of one cycle at 95 °C for 2 min; 30 cycles of 95 °C for 20 seconds, 58 °C for 10 seconds and 70 °C for 1 minute.

Constructs were expressed through inoculation, followed by inoculation with TSWV after 35 - 60 hours, preferably 48h. Inoculated leaves were harvested between 4-6, preferably 5 days after inoculation for RNA extraction.

Constructs limited TSWV genomic RNA accumulation and propagation compared to no construct-inoculated plants.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

### REFERENCES

Cardoso JMS, Félix MR, Clara MIE, Oliveira S (2005) The complete genome sequence of a new necrovirus isolated from Olea europaea L. Archives of Virology 150:815-823
Edwarson JR, Christie RG (1986) Tomato spotted wilt virus. Viruses infecting forage legumes. , Vol Vol. III
Varanda CMR, Félix MR, Soares CM, Oliveira S, Clara MIE (2011a) Specific amino acids of Olive mild mosaic virus coat protein are involved on transmission by Olpidium brassicae. Journal of General Virology 92:2209-
Cardoso, J. M. S., Félix, M. R., Clara, M. I. E. & Oliveira, S. (2005). The complete genome sequence of a new necrovirus isolated from Olea europaea L. Arch Virol 150, 815-823.
Pires, P.; Dandlen, S. A.; Nolasco, G.; Félix, M. R.; Materatski, P.; Varanda, C. M. R.; Marques, N. (2022). Expression of Tomato spotted wilt virus genes in antisense orientation affects virus progression in Nicotiana benthamiana. Paper presented in 16th Congress of the Mediterranean Phytopathological Union (MPU), 2022.
Pires, V.; A. Dandlen, S. A.; Nolasco, G.; Felix, M.d.R.; Materatski, P.; Varanda, C.M.R.; Marques, N. (2021). Tomato spotted wilt virus genes expressed in antisense orientation and their ability to control virus progression in Nicotiana benthamiana. Paper presented in VI PhD Students Meeting in Environment and Agriculture, 2021.
Varanda, C.M.R.; Félix, M.R.; Soares, C.M.; Oliveira, S.; Clara, M.I.E. (2011). Specific amino acids of Olive mild mosaic virus coat protein are involved on transmission by Olpidium brassicae. Journal of General Virology, 92 (9): 2209-2213.
Varanda, C.M.R.; Materatski, P.; Campos, M.D.; Clara, M.I.E.; Nolasco, G.; Félix, M.R. (2018). Olive mild mosaic virus Coat Protein and p6 are Suppressors of RNA Silencing and Their Silencing Confers Resistance Against OMMV. Viruses, 10: 416.
Varanda, C.M.R., Félix, M.R.; Materatski, P.; Campos, M. D.; Marques, N.; Clara, M.I.; Nolasco, G. (2019). Development of a viral vector to control TSWV in tomato plants. Abstract book of the IV PhD Students Meeting in Environmental and Agriculture 11th and 12th November 2019. Pólo da Mitra, Universidade de Évora.
Varanda, C.M.R.; Félix, M.R.; Materatski, P.; Campos, M.D.; Patanita, M.; Marques, N.; Clara, M.I.; Nolasco, G. (2021). Development of OMMV as a VIGS vector for plant protection. Paper presented in XXI SPB - National Congress of Biochemistry.

## Claims

1. Isolated nucleic acid sequence having at least 95% homology with SEQ ID No 1, preferably at least 98% homology with SEQ ID No 1, even more preferably having at least 99% homology with SEQ ID No 1, the isolated sequence comprising at least one point mutation selected from: cytosine to guanine at nt 3361, guanine to cytosine at nt 3365 and/or guanine to thymine at nt 3368.

2. Isolated sequence according to the previous claim wherein the sequence is a partial sequence of the non-structural protein NSm from Tomato Spotted Wilt Virus (TSWV).

3. Vector comprising the isolated sequence of any of the previous claims.

4. Construct comprising the isolated sequence or vector according to any of the previous claims and a partial isolated sequence of TSWV in antisense orientation.

5. Construct comprising the isolated sequence or vector according to claims 1-3 and a further isolated sequence having at least 95% homology with SEQ ID No 2, preferably at least 98% homology with SEQ ID No 2, even more preferably having at least 99% homology with SEQ ID No 2.

6. Construct according to claim 4-5 comprising an isolated sequence having at least 95% homology with SEQ ID No 3, preferably at least 98% homology with SEQ ID No 3, even more preferably having at least 99% homology with SEQ ID No 3.

7. Process for silencing a target gene in a plant comprising the steps of:
i) obtaining a vector or a construct according to any of the previous claims;
ii) introducing said vector or construct inside the plant, preferably by using viral suspension;
iii) inoculating the vector or construct into the plant, preferably by spraying, even more preferably by spraying with a compressor comprising an airbrush;
consequently activating the plant's specific defence mechanism, therefore silencing the target gene.

8. Process according to the previous claim wherein the plant is *Solanum lycopersicum* or *Latuca sativa L..*

9. Process for producing and maintaining the isolated sequence or vector of claims 1-3 in *Nicotiana benthamiana.*

10. Plant comprising a sequence with at least 95% homology, preferably at least 98% homology, even more preferably at least 99% homology with SEQ ID No.1, SEQ ID No 2, SEQ ID No 3 or a combination thereof, wherein the plant is not obtained exclusively by crossing and selection.

11. Plant according to the previous claim, wherein the plant is an edible berry of the plant *Solanum lycopersicum* or *Latuca sativa L..*

12. Composition, particularly a phytopharmaceutical composition, for plant treatment comprising the sequence, vector or construct of claims 1-6.

13. Composition of the previous claim for Plant disease management and/or Plant immunization.
